# EUROPEAN PATENT APPLICATION

(11) **EP 2 243 430 A1**
(43) Date of publication of application: **27.10.2010**
(21) Application number: 10158107.2
(22) Date of filing: 29.03.2010
(51) Int. Cl.: A61B 8/00, H02G 11/00

(54) **Probe holder**

(30) Priority: 22.04.2009 KR 20090035123
(71) Applicant: Medison Co., Ltd., Kangwon-do 250-875 (KR)
(72) Inventor: Shim, Jae Yoon, Seoul (KR)
(74) Representative: Schmid, Wolfgang

(57) **Abstract**

A probe holder is disclosed. The probe holder (100) includes a cable (110) connected to a probe (14), an attachment portion (120) provided to the cable (110), and a holding portion (130) to which the attachment portion (120) is detachably coupled to allow the cable (110) to be held by the holding portion (130). The probe holder (100) prevents the cable (110) connected to the probe (14) from drooping when the probe (14) is held by a probe holding member (140), thereby preventing the cable (110) from being contaminated or damaged due to contact with a floor.

## Description

### BACKGROUND OF THE INVENTION

### 1. Field of the Invention

The present invention relates to a probe holder and, more particularly, to a probe holder for holding a probe of an ultrasonic diagnostic apparatus.

### 2. Description of the Related Art

Generally, an ultrasonic diagnostic apparatus refers to a non-invasive apparatus that irradiates an ultrasound signal from a surface of a patient body towards a target internal organ beneath the body surface and obtains an image of a monolayer or blood flow in soft tissue from information in the reflected ultrasound signal (ultrasound echo-signal). The ultrasonic diagnostic apparatus has been widely used for diagnosis of the heart, the abdomen, the urinary organs, and in obstetrics and gynecology due to various merits thereof such as small size, low price, real-time image display, and high stability through elimination of radiation exposure, as compared with other image diagnostic systems, such as X-ray diagnostic systems, computerized tomography scanners (CT scanners), magnetic resonance imagers (MRIs), nuclear medicine diagnostic apparatuses, and the like.

Particularly, the ultrasonic diagnostic apparatus includes a probe which transmits an ultrasound signal to a target and receives the ultrasound echo-signal reflected therefrom to obtain an ultrasound internal image of the target.

The probe includes a transducer. The transducer transmits an ultrasound signal to the target and receives the ultrasound echo-signal reflected therefrom using a piezoelectric layer in which a piezoelectric material converts electrical signals into sound signals or vice versa through vibration thereof.

When using the apparatus for ultrasound diagnosis of a target, an operator moves or rotates the probe with one hand while keeping the probe in contact with a surface of the target to obtain an ultrasound image of the target. The probe is connected to a main body of the apparatus via a cable and is controlled by the main body to transmit the obtained ultrasound image to the main body of the apparatus.

The ultrasonic diagnostic apparatus may be provided with a probe holder. A user hangs the probe on the probe holder when not using the probe, and removes the probe from the probe holder when using the probe.

In such an ultrasonic diagnostic apparatus, the probe holder can hold only the probe and the cable connected to the probe droops from the probe holder, so that the cable partially contacts the floor and is liable to be contaminated or damaged. Therefore, there is a need to solve such a problem.

### SUMMARY OF THE INVENTION

The present invention is conceived to solve the problem of the related art, and an aspect of the invention is to provide an improved probe holder that can prevent a cable connected to a probe from drooping when the probe is held by the probe holder.

In accordance with one aspect of the invention, a probe holder includes: a cable connected to a probe; an attachment portion provided to the cable; and a holding portion to which the attachment portion is detachably coupled to allow the cable to be held by the holding portion.

At least one of the holding portion and the attachment portion may have magnetism.

The attachment portion may include a ferromagnetic substance to be attached to the holding portion.

The attachment portion may be formed with a through-hole into which the cable is inserted.

The probe holder may further include a probe holding member for holding the probe.

### BRIEF DESCRIPTION OF THE DRAWINGS

The above and other aspects, features and advantages of the invention will become apparent from the following description of exemplary embodiments given in conjunction with the accompanying drawings, in which:
Fig. 1 is a perspective view of an ultrasonic diagnostic apparatus including a probe holder according to one embodiment of the present invention; and
Figs. 2 and 3 are perspective views of the probe holder of Fig. 1.

### DETAILED DESCRIPTION OF THE EMBODIMENT

Embodiments of the invention will now be described in detail with reference to the accompanying drawings. It should be noted that the drawings are not to precise scale and may be exaggerated in thickness of lines or size of components for descriptive convenience and clarity only. Furthermore, terms used herein are defined by taking functions of the invention into account and can be changed according to the custom or intention of users or operators. Therefore, definition of the terms should be made according to overall disclosures set forth herein.

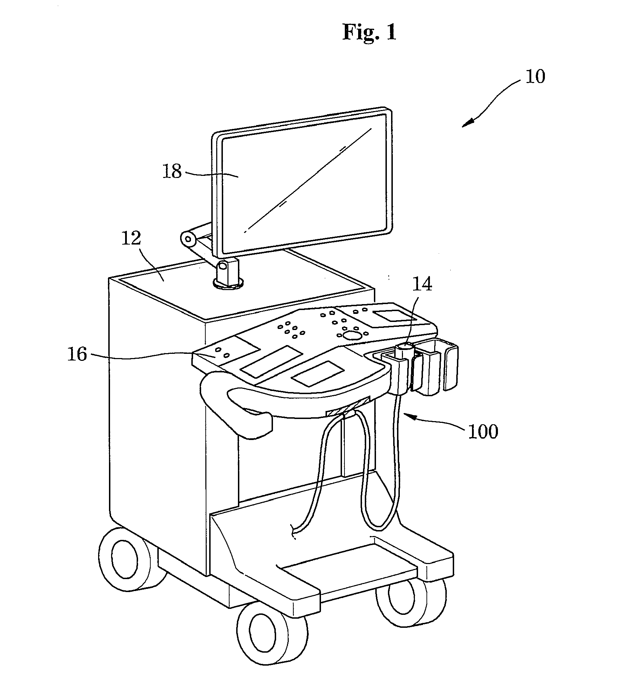

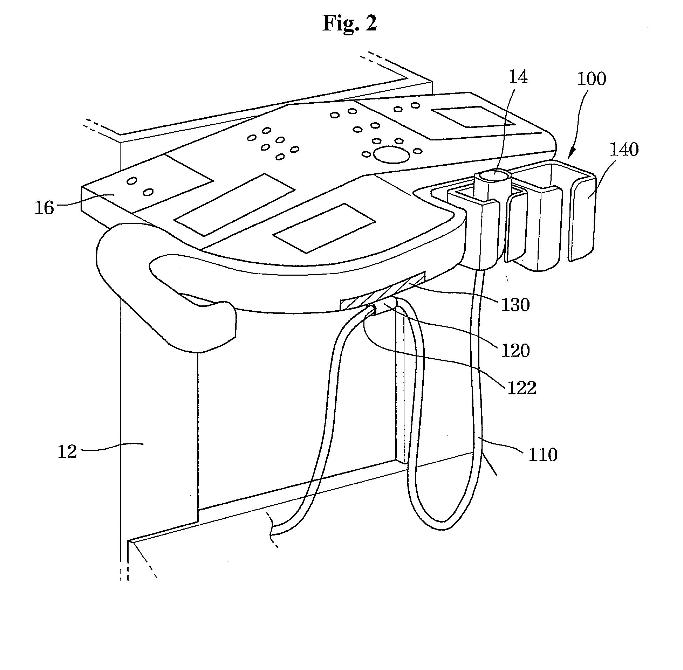

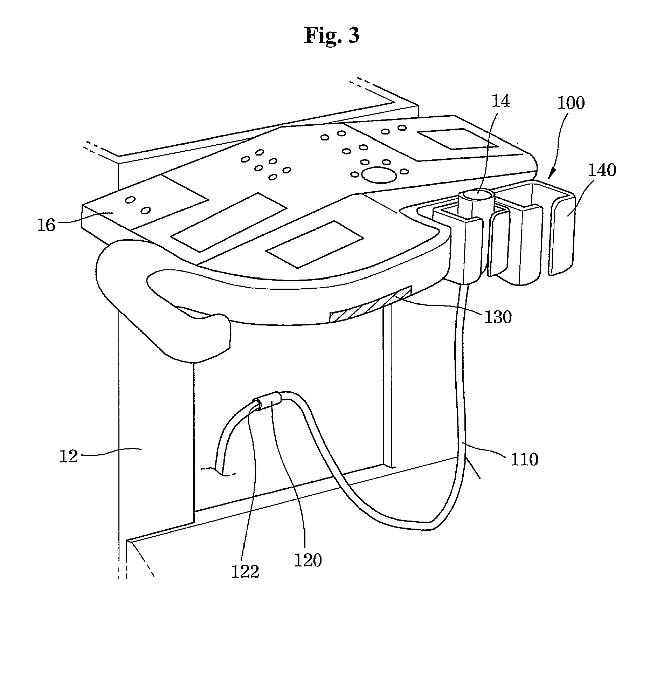

Fig. 1 is a perspective view of an ultrasonic diagnostic apparatus including a probe holder according to one embodiment of the invention, and Figs. 2 and 3 are perspective views of the probe holder of Fig. 1

Referring to Figs. 1 to 3, a probe holder 100 according to one embodiment of the invention is provided to an ultrasonic diagnostic apparatus 10. The ultrasonic diagnostic apparatus 10 includes a main body 12 that receives main components of the apparatus, a probe 14 that transmits and receives ultrasound waves, a control panel 16 that includes various switches and keys to input commands for manipulation of the apparatus, and a display unit 18 that displays images of ultrasonic diagnosis results.

In this embodiment, the probe holder 100 is illustrated as being provided to the control panel 16. However, it should be understood that the invention is not limited thereto and the probe holder 100 may be provided to any part of the ultrasonic diagnostic apparatus 10 including the control panel 16. In this embodiment, the probe holder 100 provided to the control panel 16 will be described as one example.

The probe holder 100 includes a cable 110, an attachment portion 120, and a holding portion 130.

The cable 110 is connected to the probe 14. The cable 110 is connected at one side thereof to the probe 14 and at the other side thereof to the main body 12 via a connector (not shown), so that the probe 14 is connected to the main body 12 through the cable 110.

The attachment portion 120 is provided to the cable 110. The attachment portion 120 is formed with a through-hole 122. The cable 110 is inserted into the through-hole 122 and the attachment portion 120 is coupled to the cable 110 by inserting the cable 110 into the through-hole 122. The cable 110 may be movably inserted into the through-hole 122 to be freely pulled to one side or the other.

The holding portion 130 is provided to the control panel 16. The attachment portion 120 is detachably coupled to the holding portion 130 so that the holding portion 130 holds the cable 110 inserted into the through-hole 122 of the attachment portion 120. In order to allow the attachment portion 120 and the holding portion 130 to be detachably coupled to each other, at least one of the attachment portion 120 and the holding portion 130 has magnetism. In other words, one or both of the attachment portion 120 and the holding portion 130 may have magnetism so that the attachment portion 120 can be attached to the holding portion 130. Herein, the expression "have magnetism" means that when the attachment portion 120 approaches or comes into contact with the holding portion 130, the attachment portion 120 is attached to the holding portion 130 like a magnet. For example, the entirety of the holding portion 130 may be made of a material having magnetism or a portion of the holding portion 130 to which the attachment portion 120 will be attached may be made of the material having magnetism. As such, the present invention may be realized in various modifications.

In one embodiment, the attachment portion 120 may comprise a ferromagnetic substance so as to be attached to the holding portion 130, which may comprise the material having magnetism or a material to which the ferromagnetic substance can be attached. Thus, the attachment portion 120 can be detachably attached to the holding portion 130. As a result, the cable 110 can be detachably attached to the holding portion 130 by the attachment portion 120. As described above, in the probe holder 100 according to the embodiment, the attachment portion 120 and the holding portion 130 can be easily attached to or detached from each other by magnetism, and when using the probe, a user can conveniently remove the probe 14 from the probe holder 100 merely by slightly pulling the cable 110 so that the cable can be easily attached to or detached from the holding portion.

The probe holder 100 may further include a probe holding member 140. The probe holding member 140 is provided to the probe holder 100 to hold the probe 14. The probe holding member 140 may be attached to the control panel 16 or to the main body 12 to be separated from the control panel 16.

In this embodiment, the attachment portion 120 is illustrated as comprising the ferromagnetic substance, but the present invention is not limited thereto and may be realized in various modifications. For example, both the attachment portion 120 and the holding portion 130 may comprise the material having magnetism to be detachably attached to each other. Alternatively, the attachment portion 120 may comprise the material having magnetism and the holding portion 130 may comprise the ferromagnetic substance. Alternatively, Velcro fasteners may be fixed to the attachment portion 120 and the holding portion 130.

Further, in this embodiment, the attachment portion 120 is illustrated as having a single through-hole 122, but the invention is not limited thereto. Alternatively, the attachment portion 120 may be formed with a plurality of through-holes 122 to receive a plurality of cables 110 therein.

In the probe holder 100 according to this embodiment, the attachment portion 120 is attached to the holding portion 130 so that the cable 110 coupled to the attachment portion 120 is held by the holding portion 130 and prevented from drooping when the probe holding member 140 holds the probe 14, whereby the cable 110 is prevented from being contaminated or damaged due to contact with the floor.

Furthermore, in the probe holder 100 according to this embodiment, the attachment portion 120 is detachably attached to the holding portion 130 so that the cable 110 coupled to the attachment portion 120 is detachably attached to the holding portion 130. Therefore, even when the cable 110 is held by the holding portion 130, the attachment portion 120 is separated from the holding portion 130 to allow the cable 110 to be released from the held state in the event that the cable 110 is caught by a certain object, for example, a foot of a user, thereby preventing the main body 12 or the probe 14 from being damaged by the force applied to the cable 110.

As apparent from the above description, according to the embodiment of the invention, the probe holder prevents the cable connected to the probe from drooping when holding the probe, thereby preventing the cable from being contaminated or damaged due to contact with the floor.

Further, the attachment portion can be easily attached to or detached from the holding portion using magnetism.

Thus, when using the probe, a user can conveniently remove the probe from the probe holding member merely by slightly pulling the cable without separate endeavor for releasing a holding state of the probe holder, so that the cable can be conveniently attached to or detached from the holding portion.

Furthermore, even when the cable is in a held portion, the attachment portion is separated from the holding portion to allow the cable to be released from the held state in the event that the cable is caught by a certain object, for example, a foot of a user, and is then abruptly pulled by a great force, thereby preventing the main body or probe from being damaged by the force applied to the cable.

Although some embodiments have been provided to illustrate the invention in conjunction with the drawings, it will be apparent to those skilled in the art that the embodiments are given by way of illustration only, and that various modifications and equivalent embodiments can be made without departing from the spirit and scope of the invention. The scope of the invention should be limited only by the accompanying claims.

## Claims

1. A probe holder (100), **characterized by** comprising:
a cable (110) connected to a probe (14);
an attachment portion (120) provided to the cable (110); and
a holding portion (130) to which the attachment portion (120) is detachably coupled to allow the cable (110) to be held by the holding portion (130).

2. The probe holder (100) according to claim 1, **characterized in that** at least one of the holding portion (130) and the attachment portion (120) has magnetism.

3. The probe holder (100) according to claim 1, **characterized in that** the attachment portion (120) comprises a ferromagnetic substance to be attached to the holding portion (130).

4. The probe holder (100) according to claim 1, **characterized in that** the attachment portion (120) is formed with a through-hole (122) into which the cable (110) is inserted.

5. The probe holder (100) according to any one of claims 1 to 4, **characterized by** further comprising: a probe holding member (140) for holding the probe (14).
